# EUROPEAN PATENT APPLICATION

(11) **EP 3 029 116 A1**
(43) Date of publication of application: **08.06.2016**
(21) Application number: 15195406.2
(22) Date of filing: 19.11.2015
(51) Int. Cl.: C09D 7/00

(54) **SORBIC ACID ESTER COMPOSITION**

(30) Priority: 01.12.2014 US 201462085737 P; 17.03.2015 US 201562134038 P
(71) Applicant: Dow Global Technologies LLC, Midland, MI 48674 (US); Rohm and Haas Company, Philadelphia, PA 19106-2399 (US)
(72) Inventor: ARTURO, STEVEN, COLLEGEVILLE, PA Pennsylvania 19426 (US); ARUMUGAM, SELVANATHAN, COLLEGEVILLE, PA Pennsylvania 19426 (US); ELL, JOHN, COLLEGEVILLE, PA Pennsylvania 19426 (US); EVEN, RALPH C., COLLEGEVILLE, PA Pennsylvania 19426 (US); ROWE, BRANDON, COLLEGEVILLE, PA Pennsylvania 19426 (US); SPARKS, JUSTIN, COLLEGEVILLE, PA Pennsylvania 19426 (US); YU, DECAI, MIDLAND, MI Michigan 48674 (US)
(74) Representative: Mauro, Marina Eliana

(57) **Abstract**

The present invention is a method comprising the steps of a) applying a composition comprising a curing agent and a cure modulating additive to a substrate; and b) allowing the composition to cure. The curing agent is a sorbic acid ester or a sorbamide and the cure modulating agent is a reagent capable of modulating the rate of cure of the curing agent.

## Description

### Background of the Invention

The present invention relates to a sorbic acid ester or sorbamide composition, more particularly a composition comprising a sorbic acid ester or sorbamide and an additive designed to control the cure rate of a coating composition containing a sorbic acid ester or sorbamide.

It has recently been discovered that sorbic acid esters and sorbamides are effective as high boiling, low VOC coalescents for coating compositions that have been shown to enhance film hardness and film formation at or below room temperature. A tendency of these coalescents is their propensity for curing too rapidly, thereby resulting in the formation of undesirable color bodies, or curing too slowly. It would therefore be an advance in the art coating compositions to discover a way to control the cure kinetics of sorbic acid esters and sorbamides.

### Summary of the Invention

The present invention addresses a need in the art by providing, in a first aspect, a method comprising the steps of 1) applying to a substrate a composition comprising a) a curing agent which is a sorbic acid ester or a sorbamide and b) a cure modulating additive; and 2) allowing the composition to cure; wherein the cure modulating additive is a compound functionalized with i) an S=O group; ii) a carbonyl group, iii) an amine group, iv) a hydroxyl group; v) a carboxylic acid group; vi) a sulfonic acid or sulfonate group; vii) a phosphoryl group; viii) a phosphonic acid or phosphonate group; or ix) an amide, urea, or urethane group; with the proviso that when the curing agent is a sorbic acid ester or sorbamide that is not functionalized with a primary or secondary amine group or a hydroxyl group 5 to 7 bonds removed from the oxygen atom of the sorbic acid ester or sorbamide carbonyl group, the cure modulating additive is functionalized with i) an amine group; ii) a hydroxyl group; iii) a carboxylic acid group; iv) a sulfonic acid group; v) an S=O group; or vi) a phosphorus acid group.

In a second aspect, the present invention is a composition comprising: a1) a polymer and a solvent for the polymer or a2) a dispersion of polymer particles; b) from 0.5 to 35 weight percent of a sorbic acid ester or sorbamide; and c) a cure modulating additive which is a compound functionalized with i) an S=O group; ii) a carbonyl group, iii) an amine group, iv) a hydroxyl group; v) a carboxylic acid group; vi) a sulfonic acid or sulfonate group; vii) a phosphoryl group; viii) a phosphonic acid or phosphonate group; or ix) an amide, urea, or urethane group; with the proviso that when the sorbic acid ester or the sorbamide is not functionalized with a primary or secondary amine group or a hydroxyl group 5 to 7 bonds removed from the oxygen atom of the sorbic acid ester or sorbamide carbonyl group, the cure modulating additive is functionalized with i) an amine group; ii) a hydroxyl group; iii) a carboxylic acid group; iv) a sulfonic acid group; an S=O group or vi) a phosphorus acid group. The composition of the present invention is useful for making coatings that can be cured efficiently without the formation of undesirable color bodies.

### Detailed Description of the Invention

In a first aspect, the present invention is a method comprising the steps of 1) applying to a substrate a composition comprising a) a curing agent which is a sorbic acid ester or a sorbamide and b) a cure modulating additive; and 2) allowing the composition to cure; wherein the cure modulating additive is a compound functionalized with i) an S=O group; ii) a carbonyl group, iii) an amine group, iv) a hydroxyl group; v) a carboxylic acid group; vi) a sulfonic acid or sulfonate group; vii) a phosphoryl group; viii) a phosphonic acid or phosphonate group; or ix) an amide, urea, or urethane group; with the proviso that when the curing agent is a sorbic acid ester or sorbamide that is not functionalized with a primary or secondary amine group or a hydroxyl group 5 to 7 bonds removed from the oxygen atom of the sorbic acid ester or sorbamide carbonyl group, the cure modulating additive is functionalized with i) an amine group; ii) a hydroxyl group; iii) a carboxylic acid group; iv) a sulfonic acid group; an S=O group or vi) a phosphorus acid group. The curing agent is preferably a liquid at 20 °C and preferably characterized by the following formula: 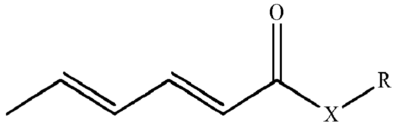 where R is a C₁-C₂₀ linear or branched alkyl group optionally functionalized with an ether, thioether, amine, hydroxyl, ester, phenyl, alkyenyl groups, or combinations thereof; and C(O)X is an ester group or an amide group.

Preferably, R is -(CH₂-CH(R¹)-O)ₙ-R²,-CH(R¹)-CH₂-O-R², or linear or branched -R³-OR²; where R¹ is H, C₁-C₆-alkyl,-CH₂OH, or phenyl;
R² is H, C₁-C₆-alkyl, benzyl, or CH₃CH=CH-CH=CH=C(O)-; allyl; -C(O)-CR⁴=CH₂;
R³ is a bivalent C₄-C₁₀-linear or branched alkyl or hydroxyalkyl group;
R⁴ is H or CH₃; and
n is 1 to 7.

The curing agent is preferably a liquid at 25 °C and preferably has a molecular weight in the range of 126 g/mol to 2000 g/mol, more preferably to 1000 g/mol, and most preferably to 500 g/mol. It is possible that the curing agent includes more than one sorbic acid ester or sorbamide groups, or combinations thereof.

The curing agent of the composition of the present invention can be prepared in a variety of ways such as those set forth in the following schemes where R is as previously defined and Y is OH or Cl:

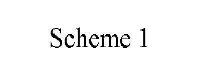

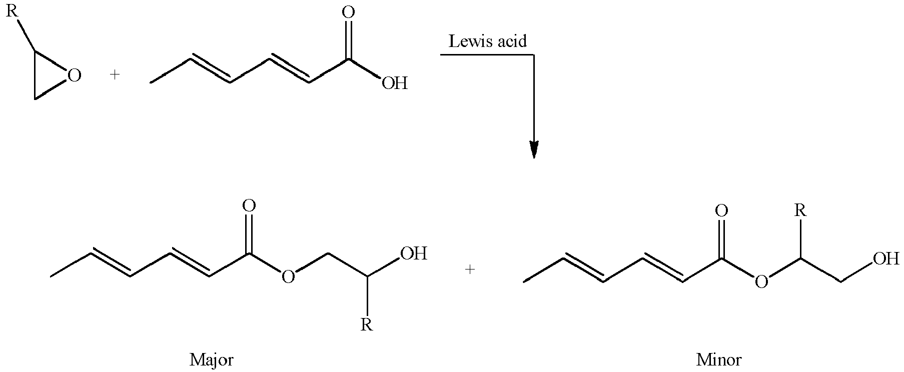

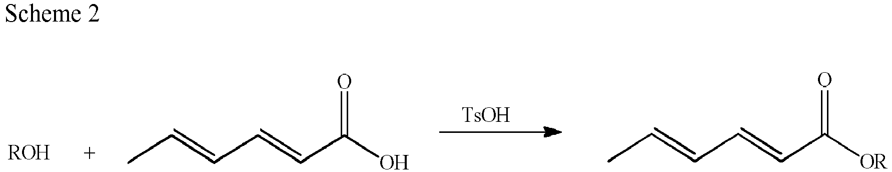

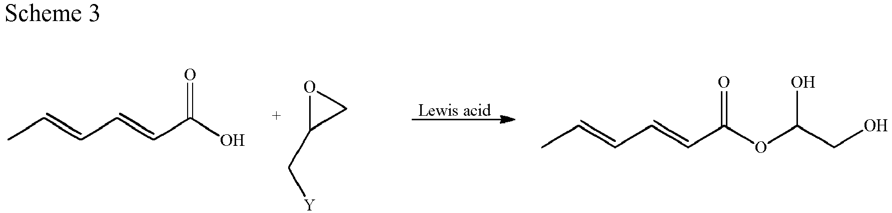

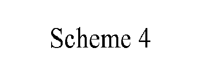

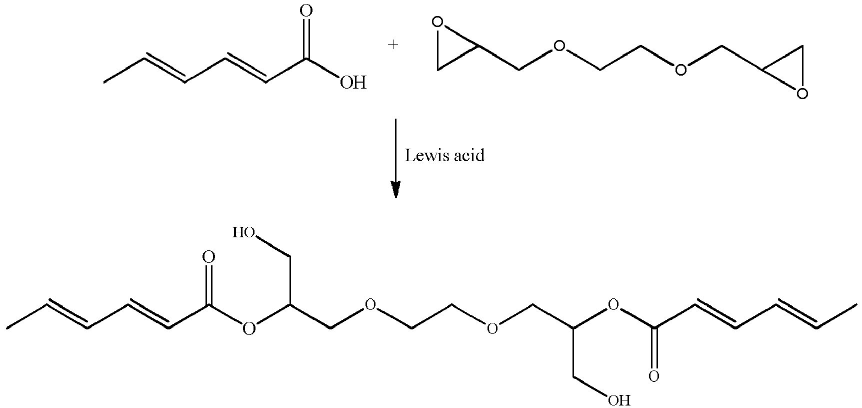

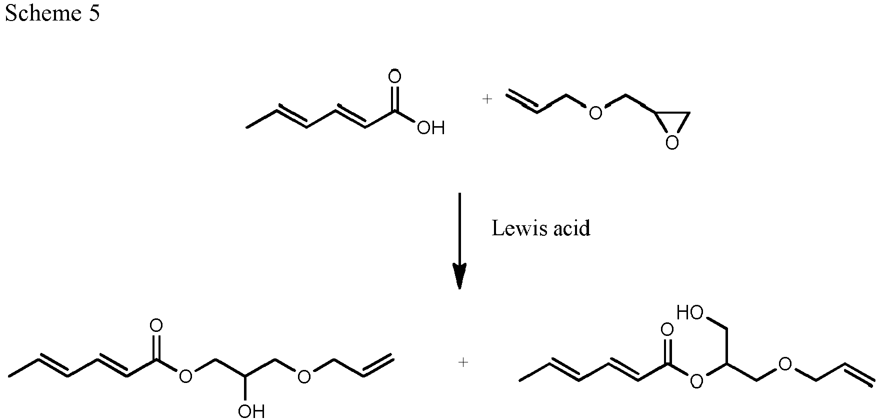

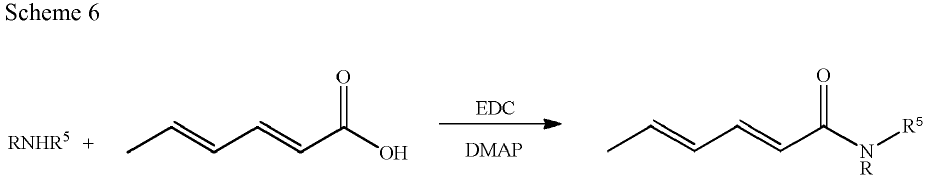

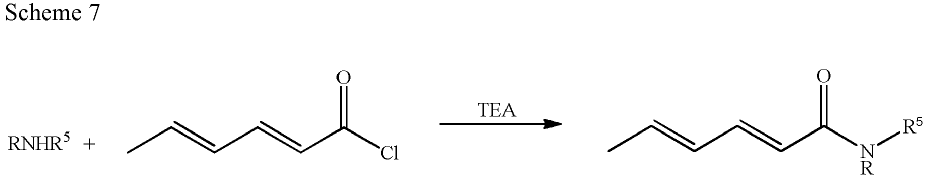

EDC is 1-ethyl-3-(3-dimethylaminopropyl)carbodiimide, DMAP is 4-dimethylamino pyridine, and TEA is triethylamine.

The curing agent is preferably a sorbate ester, which can also be prepared, for example, by way of transesterification of an alcohol and the sorbic acid or by reaction of the alcohol with an acid halide or an anhydride of the sorbic acid.

The cure modulating additive can be one that accelerates the rate of cure, one that attenuates the rate of cure, or one that can serve both functions. It has been discovered that sorbic acid esters or sorbamides cure too slowly when functionalized with a primary or secondary amine, or a primary, secondary or tertiary alcohol that is from 5 to 7, preferably 5 to 6 bonds removed from the oxygen atom of the sorbic acid ester or sorbamide carbonyl group. Examples of sorbic acid esters with an alcohol or amine that is from 5 or 6 bonds removed from the oxygen atom of the sorbic acid ester or sorbamide are illustrated:

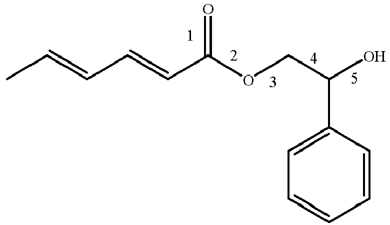

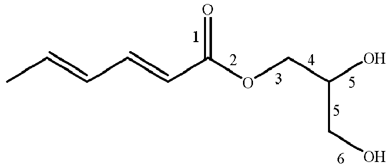

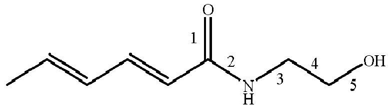

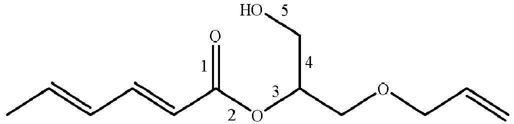

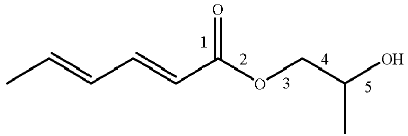

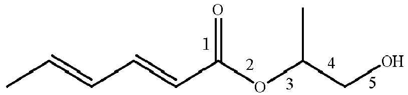

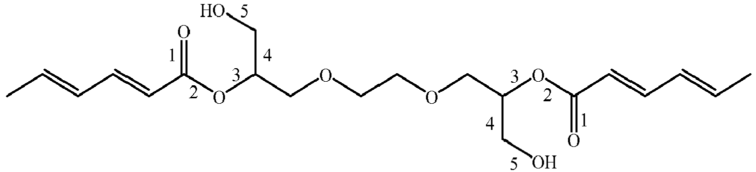

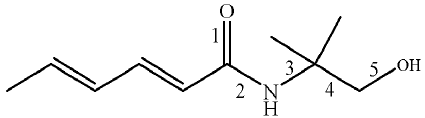

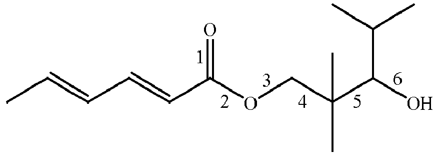

Examples of sorbic acid esters that are not functionalized with amine or alcohol group 5-7 carbon atoms removed from the oxygen atom of the sorbic acid ester or sorbamide carbonyl group are illustrated below.

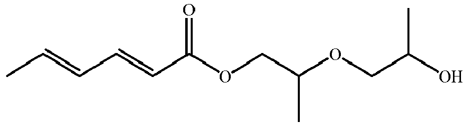

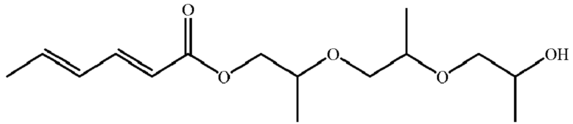

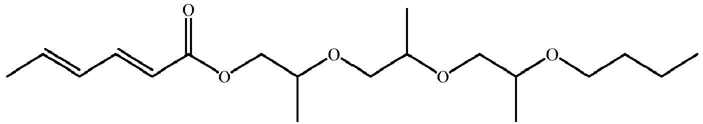

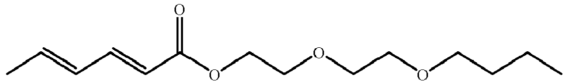

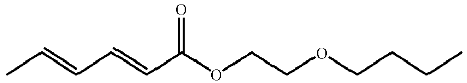

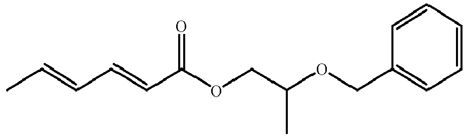

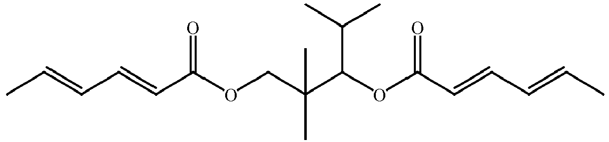

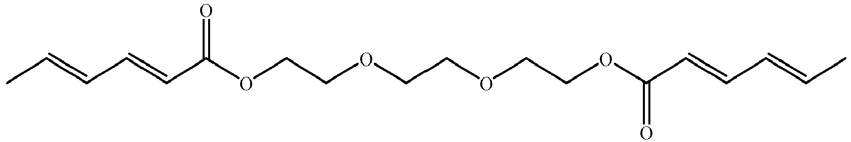

Additives that are capable of accelerating the rate of cure of sorbic acid esters and sorbamides having the alcohol or amine functionality as described above are compounds functionalized with a) an S=O group including dimethyl sulfoxide, diphenyl sulfoxide, di-4-tolyl sulfoxide, methyl phenyl sulfoxide, dibenzyl sulfoxide, dibutyl sulfoxide, or di-*t*-butyl sulfoxide; b) a carbonyl group such as methyl dodecanonate, methyl decanonate, methyl ethyl ketone, or nonaldehyde; c) an amine group such as 1-decylamine, 1-dodecylamine, diisopropylamine, tributylamine, or diisopropylethylamine; d) a hydroxyl group such as 2-ethyl-1-hexanol, 1-decanol, 3-hydroxy-2,2,4-trimethylpentyl 2-methylpropanoate (commercially available as Texanol coalescent), 2-nonanol, ethylene glycols, diethylene glycol, triethylene glycol, propylene glycol, phenol, or benzyl alcohol; e) a carboxylic acid group such as phenyl acetic acid, benzoic acid, 1-decanoic acid, or 1-octanoic acid; f) a sulfonic acid or sulfonate group such as methyl sulfonic acid, benzene sulfonic acid, 4-tolyl sulfonic acid, 1-octylsulfonic acid, 1-dodecyl sulfonic acid, methyl methanesulfonate, or methyl tosylate; g) a phosphoryl group such as triphenylphosphine oxide, tributylphosphine oxide, triethylphosphine oxide, or tribenzylphosphine oxide; h) a phosphonic acid or phosphonate group such as ethylphosphonic acid, monoethyl ethylphosphonate, butylphosphonic acid, benzylphosphonic acid, or benzenephosphonic acid; or i) an amide, urea, or urethane group such as N-ethylpropionamide, N-butyl valeramide, N, N'-diethylurea, N, N'-dibutylurea, diethylurethane, and diethylurethane.

Additives that are capable of slowing down the rate of cure of sorbic acid esters and sorbamides that do not have the aforementioned alcohol or amine functionality are compounds functionalized with an S=O group; an amine group; a hydroxyl group; a carboxylic acid group; a sulfonic acid group; or a phosphorus acid group.

Though not bound by theory, it is believed that the curing agent cures too rapidly or too slowly without the cure modulating additive because of the presence or absence of intramolecular hydrogen bonding in the curing agent. More particularly, it is believed that a hydroxyl or amine group 5-7 bonds removed from the oxygen atom of the sorbic acid ester or sorbamide group interferes with the curing mechanism; it is further believed that the addition of a so-called hydrogen-bonding acceptor (for example, a sulfoxide) disrupts intramolecular hydrogen bonding, thereby promoting curing. It is further believed that sorbic acid esters or sorbamides that do not contain amines or hydroxyl groups capable of intramolecular hydrogen bonding cure too rapidly and the cure rate for these compounds can be attenuated by interaction with the carbonyl group of the sorbic acid ester or sorbamide. Some classes of cure modulating additives can serve as either cure rate accelerators or cure rate attenuators (for example, alcohols, sulfoxides, or primary or secondary amines).

The sorbic acid ester or sorbamide is advantageously combined with the cure modulating agent in proportions that suitably control the rate of cure of the sorbic acid ester or sorbamide. Where the cure modulating contains amine or hydroxyl functionality as described hereinabove, the mole equivalents of preferably the sulfoxide, amine, or hydroxyl group of the cure modulating agent is from 0.25, more preferably from 0.5, most preferably from 0.75; to 4, more preferably to 2, most preferably to 1.25 with respect to the hydroxyl or amine groups in the curing agents. Similarly, where the cure modulating agent does not contain amine or hydroxyl functionality, the mole equivalents of preferably the sulfoxide or hydroxyl groups is from 0.25, more preferably from 0.5, most preferably from 0.75; to 4, more preferably to 2, most preferably to 1.25 with respect to the carbonyl groups in the curing agent. By way of illustration, the following compound has two equivalents of OH groups per molecule and would therefore require twice as many moles of a compound containing a single hydrogen acceptor group, such as dimethyl sulfoxide, to achieve the same mole equivalents:

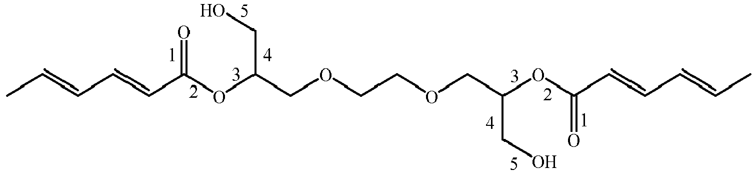

In another aspect, the present invention is a composition comprising: a1) a polymer and a solvent for the polymer or a2) an aqueous dispersion of polymer particles; b) from 0.5 to 35 weight percent of a curing agent which is a sorbic acid ester or sorbamide; and c) a cure modulating additive, which is a compound functionalized with i) an S=O group; ii) a carbonyl group, iii) an amine group, iv) a hydroxyl group; v) a carboxylic acid group; vi) a sulfonic acid or sulfonate group; vii) a phosphoryl group; viii) a phosphonic acid or phosphonate group; or ix) an amide, urea, or urethane group; with the proviso that when the sorbic acid ester or the sorbamide is not functionalized with a primary or secondary amine group or a hydroxyl group 5 to 7 bonds removed from the oxygen atom of the sorbic acid ester or sorbamide carbonyl group, the cure modulating additive is functionalized with i) an amine group; ii) a hydroxyl group; iii) a carboxylic acid group; iv) a sulfonic acid group; an S=O group or vi) a phosphorus acid group.

The composition comprises a1) a polymer and a solvent for the polymer, which is a suitable organic solvent such as toluene, xylene, or mesitylene, or a2) an aqueous dispersion of polymer particles. Preferably, the composition comprises an aqueous dispersion of polymer particles (a latex). The curing agent, preferably the sorbate ester, is preferably imbibed into the polymer particles. As used herein, the word "imbibed" means that at least 60% of the coalescent in the composition is incorporated into the polymer particles, that is, less than 40% of the coalescent is present in the aqueous phase of the latex. Preferably, at least 90%, more preferably at least 95, and most preferably at least 98% of the coalescent is imbibed into the polymer particles. The extent of imbibing can be determined by proton NMR spectroscopy, as follows: In a first experiment, a latex containing the curing agent is placed as is in an NMR spectroscopy tube and resonances associated with the coalescent are monitored in the aqueous phase of the emulsion latex. Under this condition, signals from the aqueous phase are the only ones detected because the molecules in the latex particles are partly immobilized, leading to extremely broad signals that are not detected within the spectral width for aqueous phase materials. The spectra reveals only slight traces of the curing agent (<1% by weight) in the aqueous phase. In contrast, sorbic acid can be detected quantitatively or nearly quantitatively in the aqueous phase, which demonstrates that it does not partition into the latex particles.

In a second independent NMR spectroscopic test to demonstrate imbibing of the coalescent, a broadline proton resonance is monitored for molecules in the latex particles by varying the concentration of the coalescent in the latex from 0 to 16% weight, based on the weight of the latex. As the amount of the coalescent is increased, the linewidth narrows linearly, which corresponds to a reduction of the T_{g} of the polymer or an increase in the polymer dynamics of the polymers in the particles due to the increase in the curing agent concentration. The narrowing of linewidth of the resonances associated with the polymer in the particles also directly correlates with minimum film formation of the films arising from these emulsions.

The curing agent, preferably the sorbate ester, is preferably used at a concentration in the range of from 1 to 20, more preferably to 12 weight percent, based on the weight of the polymer particles and the sorbic acid ester or sorbamide. Examples of suitable aqueous dispersions of polymer particles (also known as latexes) include acrylic, styrene-acrylic, vinyl ester-acrylic, polyurethane, alkyd, and vinyl-ester polyethylene latexes. The solids content of the latex is preferably in the range of 30 to 60%, and the T_{g} of the polymer particles, as calculated using the Fox equation, is preferably in the range of from 0 °C, more preferably from 20 °C, to 100 °C, more preferably to 60 °C.

The composition may be pigmented or non-pigmented. A preferred pigmented coating contains TiO₂. The polymer particles may also include structural units of other monomers, particularly a post-crosslinking monomer (that is, a monomer that causes significant crosslinking after onset of film formation of the composition when applied to a substrate). Examples of suitable post-crosslinking monomers include acetoacetoxyethyl methacrylate (AAEM) and diacetone acrylamide (DAM).

Additionally, the composition advantageously further includes one or more of the following materials: rheology modifiers; opaque polymers; fillers; colorants; pigments, including encapsulated or partially encapsulated pigments; dispersants; wetting aids; dispersing aids; anti-oxidants; dispersant adjuvants; chelating agents; surfactants; co-solvents; additional coalescing agents and plasticizers; defoamers; preservatives; anti-mar additives; flow agents; leveling agents; slip additives; and neutralizing agents.

Coatings with suitable hardness can be prepared from the composition of the presentation efficiently and with a reduction of yellowing as compared to sorbic acid ester compositions that do not contain suitable cure modulating agents.

### Examples

### Examples 1-4 - General Procedure for Curing an Applied Coating of a Sorbic acid Ester and a Cure Modulating Agent

The sorbic acid ester (1 g) was placed in a vial followed by addition of the cure modulating agent (1 g). The components were mixed using a vortex mixer to achieve a homogenous solution. A thin film of sorbic acid ester (20 mil, 0.5 mL) was drawn down on a portable Diamond plug and allowed to dry at ambient condition. The amount of sorbic acid ester conversion to polymers, the measure of cure chemistry, was followed by FTIR spectroscopy at various time intervals by monitoring change in the functionality in the sorbate molecule. The yellowing of sorbate /cure modulator mixture (Table 2) was monitored by measuring the absorbance at 420 nm by Cary-50 UV-Vis Spectrophotometer.

Comparative Examples 1-2 - General Procedure for Curing an Applied Coating of a Sorbic Acid Ester and a Cure Modulating Agent Curing With No Cure Modulating Agent

A thin film of sorbic acid ester (20 mil, 0.5 mL) was drawn down on a portable Diamond plug and allowed to dry at ambient condition and curing was measured as described in Example 1.

Table 1 illustrates the effect of a cure modulating agent on the cure rate of hydroxypropyl sorbate. Texanol coalescent is 3-hydroxy-2,2,4-trimethylpentyl 2-methylpropanoate.

**Table 1 - The Effect of a Cure Modulating Agent of the Cure Rate of Hydroxypropyl Sorbate**

| **Example** | Modulator | modulator:HPS (w/w) | 100 % cure time |
|---|---|---|---|
| Comp. 1 | None | | No curing after 30 d |
| 1 | 2-Ethyl-1-hexanol | 50:50 | 4.5 d |
| 2 | Texanol coalescent | 50:50 | 10d |
| 3 | Dimethyl Sulfoxide | 50:50 | 3.5 d |

The data illustrate that the inclusion of the rate accelerating cure modulating agent dramatically increase the rate of cure, with dimethyl sulfoxide being particularly effective.

Table 2 illustrates the effect of a cure modulating agent on the deceleration of the cure rate of ethyl sorbate (ES). The % conversion refers to the conversion of the ethyl sorbate at 10 h and % yellowing is normalized to Comparative Example 2 at 100.

**Table 2 - The Effect of a Cure Modulating Agent of the Cure Rate of Ethyl Sorbate**

| **Example** | Modulator | modulator:ES (w/w) | % Conversion @ 10h | % Yellowing after 4 wks |
|---|---|---|---|---|
| Comp. 2 | None | | 99 | 100 |
| 4 | 2-nonanol | 50:50 | 40 | 23 |
| 5 | Texanol coalescent | 50:50 | 56 | 19 |

The data show that the cure modulating agent slows down the rate of conversion and concomitantly reduces yellowing of the cured ethyl sorbate.

## Claims

1. A method comprising the steps of 1) applying to a substrate a composition comprising a) curing agent which is a sorbic acid ester or a sorbamide and b) a cure modulating additive; and 2) allowing the composition to cure; wherein the cure modulating additive is a compound functionalized with i) an S=O group; ii) a carbonyl group, iii) an amine group, iv) a hydroxyl group; v) a carboxylic acid group; vi) a sulfonic acid or sulfonate group; vii) a phosphoryl group; viii) a phosphonic acid or phosphonate group; or ix) an amide, urea, or urethane group; with the proviso that when the curing agent is a sorbic acid ester or sorbamide that is not functionalized with a primary or secondary amine group or a hydroxyl group 5 to 7 bonds removed from the oxygen atom of the sorbic acid ester or sorbamide carbonyl group, the cure modulating additive is functionalized with i) an amine group; ii) a hydroxyl group; iii) a carboxylic acid group; iv) a sulfonic acid group; v) an S=O group; or vi) a phosphorus acid group.

2. The method of Claim 1 wherein the curing agent is a sorbic acid ester functionalized with a primary or secondary amine group or a hydroxyl group 5 to 7 bonds removed from the oxygen atom of the sorbic acid ester or sorbamide carbonyl group.

3. The method of Claim 2 wherein the cure modulating additive is a liquid at 25 °C and is functionalized with an S=O group, a hydroxyl group, or an amine group; and wherein the mole equivalents of S=O, hydroxyl, or amine groups of the cure modulating additive to the curing agent is from 0.25 to 4.

4. The method of Claim 3 wherein the curing agent is selected from the group consisting of: 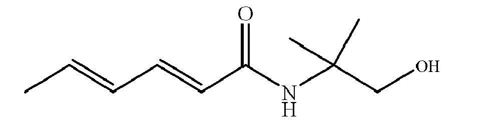 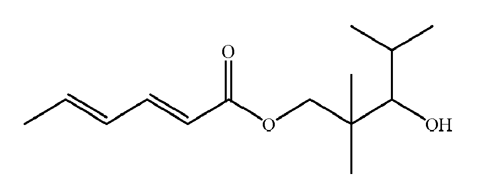 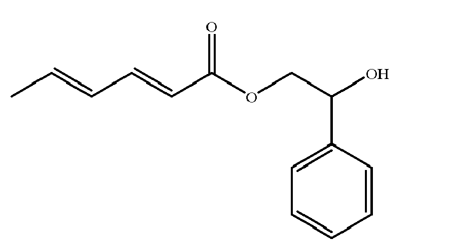 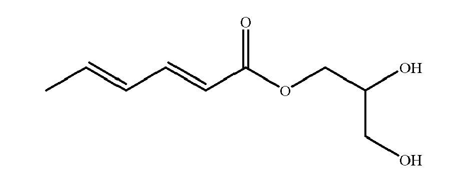 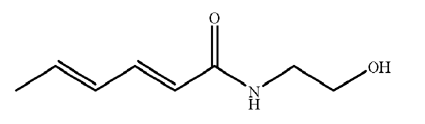 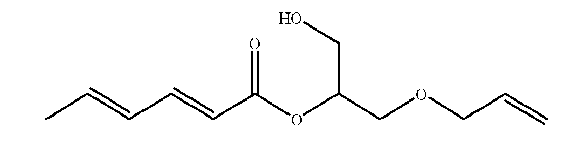 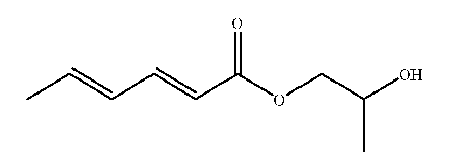 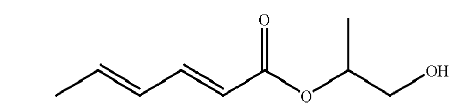 and 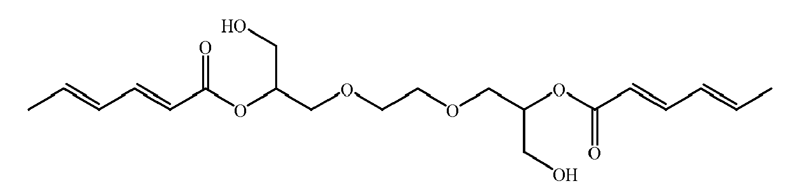 and wherein the cure modulating additive is dimethyl sulfoxide, 2-ethyl-1-hexanol, or nonanol; and wherein the cure modulating additive is dimethyl sulfoxide; 2-ethyl-1-hexanol; diethylene glycol butyl ether; or dipropylene glycol methyl ether; or 2-nonanol; and wherein the mole equivalents of the cure modulating additive to the curing agent is from 0.5 to 2.

5. The method of Claim 1 wherein the curing agent is a sorbic acid ester not functionalized with a primary or secondary amine group or a hydroxyl group that is from 5 to 7 bonds removed from the oxygen atom of the sorbic acid ester or sorbamide carbonyl group, wherein the cure modulating additive is a liquid at 25 °C and is a primary or secondary alcohol or a sulfoxide; wherein the mole equivalents of sulfoxide or hydroxyl groups of the cure modulating additive to the curing agent is from 0.25 to 4..

6. The method of Claim 5 wherein the curing agent is selected from the group consisting of: 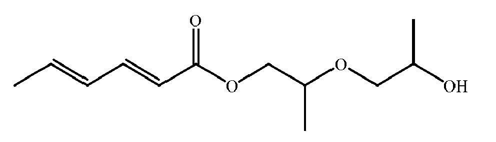 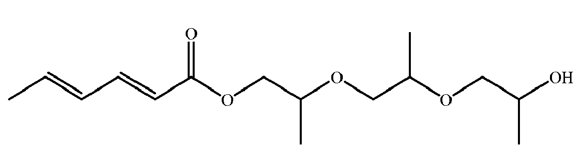 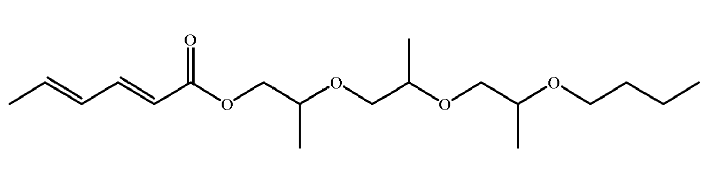 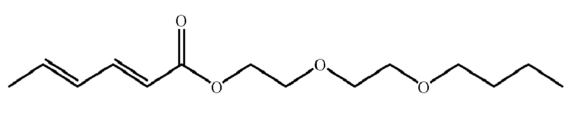 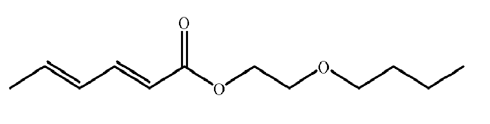 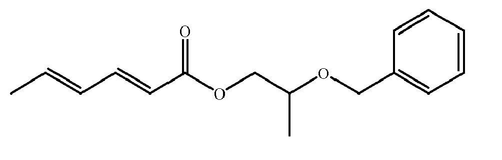 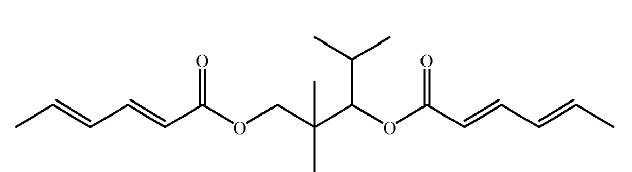 and 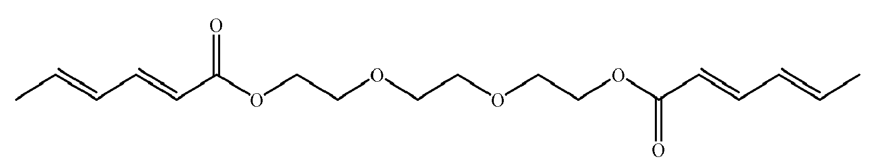 wherein the cure modulating additive is dimethyl sulfoxide; 2-ethyl-1-hexanol; diethylene glycol butyl ether; dipropylene glycol methyl ether; 3-hydroxy-2,2,4-trimethylpentyl 2-methylpropanoate; or 2-nonanol; and wherein the mole equivalents of the cure modulating additive to the curing agent is from 0.5 to 2.

7. The method of any of Claims 1 to 6 wherein the composition further comprises an aqueous dispersion of polymer particles and at least one component selected from the group consisting of a pigment, a defoamer, a surfactant, a dispersant, a rheology modifier, and a neutralizing agent.

8. The method of any of Claims 1 to 6 wherein the composition further comprises an aqueous dispersion of polymer particles, a pigment, a defoamer, a surfactant, a dispersant, a rheology modifier, and a neutralizing agent.
